# EUROPEAN PATENT SPECIFICATION

(11) **EP 2 694 205 B1**
(45) Date of publication and mention of the grant of the patent: **27.07.2022**
(21) Application number: 11713636.6
(22) Date of filing: 01.04.2011
(51) Int. Cl.: B01J 23/883, B01J 23/89, B01J 37/02, B01J 37/08, B01J 37/18, C07C 1/04, C07C 31/04, C07C 31/08, C07C 31/10, C07C 31/12, C10G 2/00, B01J 23/00

(54) **CATALYSTS FOR THE CONVERSION OF SYNTHESIS GAS TO ALCOHOLS**
KATALYSATOREN ZUR UMWANDLUNG VON SYNTHESEGAS IN ALKOHOLE
CATALYSEURS POUR LA CONVERSION DE GAZ DE SYNTHÈSE EN ALCOOLS

(43) Date of publication of application: 12.02.2014
(73) Proprietor: Dow Global Technologies LLC, Midland, MI 48674 (US)
(72) Inventor: MILLAR, Dean M., Midland MI 48640 (US); MCADON, Mark H., Midland MI 48642-3389 (US); GULOTTY, Robert J. Jr., Glendale AZ 85308 (US); BARTON, David G., Midland MI 48642 (US); FERRARI, Daniela, 2050 Antwerp (BE); BARDIN, Billy B., Lake Jackson TX 77566 (US); LIU, Yu, Lake Jackson TX 77566 (US)
(74) Representative: Beck Greener LLP
(86) International application number: PCT/US2011/030915
(87) International publication number: WO 2012/134493

(56) References cited:
- EP-A1- 1 452 231
- GB-A- 1 495 011
- US-A- 5 834 394
- CHUN-HUI MA ET AL: "MWCNT-Supported Niâ Moâ K Catalyst for Higher Alcohol Synthesis from Syngas", CATALYSIS LETTERS, KLUWER ACADEMIC PUBLISHERS-PLENUM PUBLISHERS, NE, vol. 137, no. 3-4, 22 April 2010 (2010-04-22), pages 171-179, XP019815188, ISSN: 1572-879X

## Description

In various aspects, this invention relates generally to a catalyst for converting synthesis gas (or "syngas", a mixture of gases consisting mainly of carbon monoxide (CO) and hydrogen (H₂)) into a mixture of alcohols (e.g., ethanol (EtOH), propanol (PrOH), and butanol (BuOH), optionally in conjunction with higher alcohols). In various aspects, the invention relates particularly to such a catalyst comprising a combination of nickel, molybdenum, and at least (≥) one metal selected from a group consisting of palladium, ruthenium, chromium, gold, zirconium, and aluminum, wherein said at least one metal comprises a combination of palladium and aluminum, or ruthenium and chromium, or gold and aluminum. The catalyst is preferably promoted with an alkali or alkaline earth series metal. In another aspect the invention relates to a synthesis gas conversion catalyst comprising nickel, molybdenum, gold, and chromium, a catalyst support that is silica or alpha-alumina, and a promoter that is cesium.

Ethanol and mixtures of alcohols including ethanol are used as fuels and fuel additives in place of at least a portion of petroleum-based products such as gasoline, thereby reducing the need for petroleum. The substitution of alcohols for petroleum-based fuels and fuel additives can conserve natural resources and improve environmental quality, especially when alcohols are produced from feedstocks other than petroleum, such as biomass or natural gas. Ethanol and mixtures of alcohols including ethanol can also be converted into useful chemical industry feedstock olefins, such as ethylene (C₂H₆) and propylene (C₃H₈).

Conversion of syngas to produce organic compounds has been known for many years. Some of the most useful processes are those for direct conversion of syngas to alcohols. Such conversion typically employs heterogeneous catalysts.

United States Patent (US) 4,762,858 teaches use of a molybdenum-containing catalyst that consists essentially of molybdenum, ≥ one metal selected from among thorium, yttrium, lanthanum, gadolinium, and praseodymium, and optionally ≥ one alkali or alkaline earth metal on a support to convert syngas into mixed alcohols containing ethanol and propanol.

Ma, CH., Li, HY., Lin, GD. et al. MWCNT-Supported Ni-Mo-K Catalyst for Higher Alcohol Synthesis from Syngas. Catal Lett 137, 171-179 (2010) is a journal article relating to a type of novel MWCNT-supported Ni-Mo-K catalysts for higher alcohol synthesis (HAS) from syngas that was developed.

In some aspects this invention is a syngas conversion catalyst that comprises:
(i) nickel, molybdenum, at least one metal selected from a group consisting of palladium, ruthenium, chromium, gold, zirconium, and aluminum; wherein said at least one metal comprises a combination of palladium and aluminum, or ruthenium and chromium, or gold and aluminum,
   a promoter comprising at least one of an alkali metal or alkaline earth metal, wherein each promoter is present as a metal, hydroxide, nitride, carbide, or as a salt or a combination thereof,
   and
   a catalyst support selected from a group consisting of silica, alumina, and magnesium oxide, or a mixture thereof;
      or
(ii) nickel, molybdenum, gold, and chromium, a catalyst support that is silica or alpha-alumina, and a promoter that is cesium;
and wherein the catalyst support is present in an amount that is equal to or greater than 80 weight percent based upon the total mass of all catalyst components.

The invention provides a process for producing one or more C₂-C₄ alcohols, which method comprises placing synthesis gas in contact with the catalyst of any of Claims 1-5 under conditions sufficient to convert at least a portion of the synthesis gas to at least one of ethanol, propanol and butanol, wherein the conversion temperature is from 200 to 500°C.

Preferably the catalyst is reduced using a reducing agent prior to contact with the synthesis gas.

It is preferred that at least a portion of the synthesis gas is converted to methanol.

It is preferred that at least a portion of the synthesis gas is converted to acetaldehyde.

Preferably the conditions include a minimum pressure of 500 psig (3.4 MPa).

"C₂-C₄ alcohols" means one or more alcohols selected from ethanol, propanol, and butanol, including all known isomers of such compounds.

Each syngas conversion catalyst metal may be present in free or combined form. "In free or combined form" means that a metal may be present as a free (or base) metal, an alloy, a compound, an adduct or a combination thereof. Representative compounds include hydroxides, oxides, sulfates, halides, carbides, cyanides, nitrides, nitrates, phosphates, borides, silicides, silicates, oxyhalides, carboxylates (e.g., acetates and acetylacetates), oxalates, carbonates, carbonyls, hydrides, metal-bridged and cluster compounds, and compounds where the metal is part of an anionic or cationic species. Adducts are chemical addition products of two or more distinct molecules.

For any metal, report content as a weight percent (wt%) calculated by taking a ratio of the mass of free metal content of the metal, as a numerator, and mass of all catalyst components as a denominator. Nickel, molybdenum, and ≥ one metal selected from the group consisting of palladium, ruthenium, chromium, gold, zirconium, and aluminum together are each generally present at lower limits of 0.1 wt%, more preferably 0.25 wt%, most preferably 0.5 wt% and especially 1 wt%. Upper catalyst metal limits are 50 wt%, more preferably 25 wt%, most preferably 10 wt% and especially 8 wt%, each wt% being based upon total free metal content of the catalyst.

Divide number of moles of ≥ one metal selected from the group consisting of palladium, ruthenium, chromium, gold, zirconium, and aluminum or mixtures thereof by number of moles of nickel to provide a molar ratio thereof. Preferred molar ratios range from a lower level of 1 to 200, more preferably 1 to 1 to an upper level of 8 to 1, more preferably 4 to 1.

Alkali metals include lithium, sodium, potassium, rubidium and cesium. Alkaline earth metals include beryllium, magnesium, calcium, strontium and barium. Cesium represents a preferred promoter, either alone or in combination with calcium.

Divide number of moles of nickel, molybdenum, and ≥ one metal selected from the group consisting of palladium, ruthenium, chromium, gold, zirconium, and aluminum or mixtures thereof by number of moles of alkali and alkaline earth metals to provide a molar ratio thereof. Preferred molar ratios range from a lower level of 1 to 10, more preferably 1 to 3, to an upper level of 10 to 1, more preferably 5 to 1.

The promoter is present as a metal, hydroxide, nitride, carbide or as a salt or a combination thereof. The promoter can be incorporated during synthesis gas conversion catalyst preparation by any of a wide variety of ways, such as incipient wetness, dip coating or co-precipitation.

Suitable catalyst supports include silica, alpha-alumina, magnesium oxide, carbon, chromium oxide, titanium oxide, zirconium oxide, and zinc oxide. The catalyst support is present in an amount that is ≥ 80 wt%, more preferably ≥ 90 wt% up to (≤) 99 wt%, more preferably ≤ 96wt%, each wt% being based upon total mass of all catalyst components.

The synthesis gas conversion catalyst can be prepared by a variety of methods known in the art that result in intimate contact among such components, such as incipient wetness (*see generally* ROBERT L. AUGUSTINE, HETEROGENEOUS CATALYSIS FOR THE SYNTHETIC CHEMIST 184-88 (Marcel Dekker 1996). With incipient wetness, choose sources for catalyst metals to be dispersed on the support from a variety of art-recognized water-soluble or solvent-soluble salts of the metals. Dissolve soluble salt(s) in a quantity of solvent (aqueous, non-aqueous or a combination thereof) to provide a solution; and add sufficient solution to wet, but do no more than fully saturate, the support. Evaporate solvent by applying heat, optionally under vacuum, to leave salt dispersed on the support. Repeat as necessary.

In one embodiment, prepare the synthesis gas conversion catalyst by reducing an initially prepared catalyst composition (formed by combining the nickel; molybdenum; ≥ one metal selected from the group consisting of palladium, ruthenium, chromium, gold, zirconium, and aluminum; promoter; and support using the incipient wetness technique) in a reducing atmosphere by flowing a reducing agent such as hydrogen (H₂) between ambient pressure to moderately elevated pressure (e.g., from 14.7 pounds per square inch gauge (psig) (0.10 megapascals (MPa)) to 600 psig (4.14 MPa)). Such hydrogen treatment has a lower temperature limit of 250 degrees Celsius (°C), more preferably 330 °C. The hydrogen treatment has an upper limit of 1200 °C, more preferably 700 °C. Repeat the wetting, evaporating and heating steps as needed to achieve a desired concentration of catalytic metal species or promoter on the support.

Use the syngas conversion catalyst in a fixed bed, moving bed, fluidized bed, ebullated bed or a graded bed wherein catalyst concentration or activity varies from inlet to outlet in similar manner to known catalysts. Use the catalyst either as a powder or as a shaped form.

The products of the reaction of CO and H₂ catalyzed by the syngas conversion catalyst include a mixture of C₂-C₄ alcohols, optionally in conjunction with higher alcohols; other products may include methanol, oxygenated organic compounds (oxygenates), hydrocarbons and CO₂. Report product selectivity relative to CO in mole percent (mole%) by carbon atom. For example, for a reaction that converts one mole of CO to 0.2 moles methanol, 0.1 moles ethanol, 0.067 moles *n*-propanol, 0.2 moles methane, and 0.2 moles carbon dioxide (CO₂) (and 0.233 moles of other products), report the selectivity as 20 mole% for each of these five reaction products.

Selectivity to C₂-C₄ alcohols is preferably higher than selectivity to methanol. In one embodiment, selectivity to methanol is less than one-half selectivity to C₂-C₄ alcohols. In a second embodiment, selectivity to methanol is less than one-fourth selectivity to the C₂-C₄ alcohols. Preferably only small portions of other oxygenates besides alcohols, such as ethers, carboxylic acids, esters, ketones, aldehydes, and peroxides, are formed during syngas conversion. Aldehydes may be hydrogenated to alcohols. In one variation, acetaldehyde can be hydrogenated to ethanol. In another variation, the selectivity to the desired C₂-C₄ alcohols product is 20% or above.

Obtaining these selectivity values is generally a matter of varying process conditions and catalyst composition. For example, to increase conversion within the preferred ranges using a reduced catalyst prepared as described above, one may vary one or more of temperature, pressure, gas hourly space velocity (GHSV) and syngas composition to produce a desired result. As conversion increases, product distribution of mixed alcohols produced usually shifts toward higher molecular weight alcohols. Varying recycle ratio and monitoring recycled components may also alter selectivity. For example, to obtain more C₂-C₄ alcohols in relation to methanol, methanol may be recycled or added to the syngas feed. Varying the catalyst metals themselves may provide the desired selectivity. For example, catalysts comprised of Ni-Mo-Pd-Al-Cs and Ni-Mo-Au-Cr-Cs on alpha-alumina (α-Al₂O₃) produce 28 wt% and 19 wt% ethanol selectivity (based on carbon), respectively, under the same operating conditions wherein the syngas consists of 95 mole percent or above elemental H₂ and CO gases at an H₂/CO molar ratio of 1.0, GHSV corrected to standard temperature and pressure (STP) of 4500 hour⁻¹, temperature of 300 °C, and pressure of 1500 psig (10.39 MPa), with no recycle.

Generally, selectivity to alcohols depends on pressure. In normal operating ranges, the higher the pressure at a given temperature, the more selective the process will be to mixed alcohols. Operating pressures include pressures ≥ 150 psig (1.03 MPa)) or, with pressures in excess of (>) 500 psig (3.44 MPa) being preferred and pressures > 750 psig (5.17 MPa) being more preferred. An especially preferred pressure lies within a range of from 1,500 psig (10.3 MPa) to 4,000 psig (27.6 MPa). Pressures in excess of 4,000 psig (27.6 MPa), while possible, tend to be economically unattractive due to the cost of high pressure vessels, compressors, and energy costs. With that in mind, pressures as high as 20,000 psig (137.9 MPa) are feasible, but a pressure of 10,000 psig (68.9 MPa) or less is preferred and a pressure of 5,000 psig (34.5 MPa) is still more preferred and a pressure of 2,000 psig (13.8 MPa) to 3,000 psig (20.7 MPa) provides very satisfactory results.

Temperatures used in converting syngas to mixed alcohols preferably range from a minimum of 200 °C to a maximum of 500 °C. The maximum temperature is more preferably 400 °C, still more preferably 370 °C. An especially preferred range of operation is from 240 °C to 350 °C.

The GHSV of the synthesis gas feed is a measure of the volume of H₂ plus CO gas at STP passing a given volume of catalyst in one hour. The GHSV is sufficient to produce mixed alcohols and may vary over a very wide range, preferably from 50 hour⁻¹ to 20,000 hour⁻¹. The GHSV is more preferably ≥ 2000 hour⁻¹, and still preferably ≥ 3000 hour⁻¹, but less than or equal to (≤) 10,000 hour⁻¹,more preferably ≤ 7,500 hour⁻¹. Within the preferred ranges, conversion of syngas usually decreases as GHSV increases. Concurrently, however, productivity usually increases. Measure productivity by mass of product produced per unit volume of catalyst.

At least a portion of unconverted H₂ and CO in effluent gas from the reaction may be recycled to a reactor. Express recycle amount as a ratio of moles of gas in the recycle stream to the moles of gas in a fresh feed stream. Recycle ratios may vary from zero to any number which results in the formation of a mixed alcohol product. A recycle ratio of zero is within the scope of the invention with at least some recycle preferred. After separation of the desired alcohols, if at least a portion of the effluent gas is recycled and it contains unconverted H₂ and CO, it is preferable to remove water, CO₂, and even more preferably any hydrocarbons formed. The recycle of methanol may favor production of C₂-C₄ mixed alcohols. In another variation, one or more C₂-C₄ alcohols or other alcohols may be recycled to form higher alcohols.

### EXAMPLE 1

Combine 180 milligrams (mg) gold(III) chloride hydrate (HAuCl₄·3H₂0) with 3 grams (g) silica gel (SiO₂) and 0.75 milliliters (mL) water. Heat the mixture at 70 °C for 1.5 hours under vacuum, allowing the water to evaporate to dryness. Cool the catalyst to ambient temperature. Add 67 mg nickel(II) nitrate hexahydrate (Ni(NO₃)₂·6H₂0) in 0.75 mL H₂O to the mixture. Heat the mixture at 70 °C for 1.5 hours under vacuum. Cool the catalyst to ambient temperature. Add 92 mg chromium(III) nitrate nonahydrate (Cr(NO₃)₃·9H₂O) in 0.75 mL H₂O to the mixture. Heat the mixture at 70 °C for 1.5 hours under vacuum. Cool the catalyst to ambient temperature. Add 45 mg ammonium molybdate tetrahydrate ((NH₄)₆Mo₇O₂₄·4H₂O) in 0.75 mL H₂O to the mixture. Heat the mixture at 70 °C for 1.5 hours under vacuum. Cool the catalyst to ambient temperature. Add 148 mg cesium nitrate (CsNO₃) in 0.75 mL of H₂O to the mixture. Heat for 2 hours at 70 °C under vacuum, then increase the heat in static air with a 10 °C per minute heating rate until the temperatures reaches 120 °C and hold for 1 hour. Heat to 210 °C with a 10 °C per minute heating rate and hold for 1 hour. Finally, heat to 350 °C with a 10 °C per minute heating rate and hold for 1 hour. Cool the finished catalyst to ambient temperature.

Load 0.2 mL of this catalyst into a ¼-inch (0.635 cm) stainless steel tubular reactor. Reduce the catalyst in-situ with flowing H₂ gas at 330 °C for 150 minutes. Heat the reactor and its contents to the temperature stated in Table I using an electric furnace. Use premixed H₂ and CO to pressurize the interior of the reactor to the pressure stated in Table I. The feed gas mixture contains H₂ and CO at the ratios stated in Table I. Pass the feed gas mixture at the stated GHSVs through the reactor to yield a reaction product. Pass the reaction product through a pressure letdown valve and flow past a gas chromatograph (GC) sampling point into a cooled condenser. Collect and analyze both gaseous and liquid products from the condenser. GC analysis of reaction products from the reaction chamber shows that MeOH, EtOH and other alcohols are present. Table I shows product content in terms of molar selectivity to ethanol, all alcohols (ROH selectivity), hydrocarbons and carbon dioxide together with reaction temperature and CO conversion. As used herein, "MeOH/ROH" means the fraction of alcohols that is attributed to methanol, on a molar basis by carbon atom.

**Table 1**

| | a | b | c | d | e |
|---|---|---|---|---|---|
| Temperature (°C) | 270 | 300 | 320 | 340 | 360 |
| Pressure (psig) | 1500 | 1500 | 1500 | 1500 | 1500 |
| H₂/CO (molar ratio) | 1 | 1 | 1 | 1 | 1 |
| GHSV (hour⁻¹) | 4500 | 4500 | 4500 | 4500 | 4500 |
| CO Conversion (wt%) | 0.05 | 0.2 | 0.63 | 1.4 | 20.42 |
| Ethanol selectivity (mole%) | 0 | 10 | 12 | 11 | 10 |
| ROH selectivity (mole%) | 3 | 26 | 33 | 32 | 35 |
| MeOH / ROH (molar ratio) | n/a | 0.73 | 0.48 | 0.47 | 0.4 |
| Selectivity to hydrocarbons (mole%) | 39 | 31 | 25 | 29 | 30 |
| Selectivity to CO₂ (mole%) | 58 | 42 | 41 | 39 | 40 |

### EXAMPLE 2

Replicate Example 1, but use alpha-alumina (α-Al₂O₃) instead of SiO₂. Report results in Table 2.

**Table 2**

| | a | b | c | d | e |
|---|---|---|---|---|---|
| Temperature (°C) | 270 | 300 | 320 | 340 | 360 |
| Pressure (psig) | 1500 | 1500 | 1500 | 1500 | 1500 |
| H₂/CO (molar ratio) | 1 | 1 | 1 | 1 | 1 |
| GHSV (hour⁻¹) | 4500 | 4500 | 4500 | 4500 | 4500 |
| CO Conversion (wt%) | 1 | 2.64 | 8.38 | 9.93 | 29.59 |
| Ethanol selectivity (mole%) | 16 | 19 | 19 | 10 | 7 |
| ROH selectivity (mole%) | 67 | 52 | 46 | 33 | 23 |
| MeOH / ROH (molar ratio) | 0.79 | 0.54 | 0.5 | 0.3 | 0.3 |
| Selectivity to hydrocarbons (mole%) | 22 | 25 | 29 | 34 | 39 |
| Selectivity to CO₂ (mole%) | 9 | 21 | 23 | 32 | 37 |

The data in Examples 1 and 2 show syngas conversion to alcohols even at relatively low pressures such as 500 psig (3.4 MPa) or less.

## Claims

1. A synthesis gas conversion catalyst comprising
(i) nickel, molybdenum, at least one metal selected from a group consisting of palladium, ruthenium, chromium, gold, zirconium, and aluminum; wherein said at least one metal comprises a combination of palladium and aluminum, or ruthenium and chromium, or gold and aluminum,
a promoter comprising at least one of an alkali metal or alkaline earth metal, wherein each promoter is present as a metal, hydroxide, nitride, carbide, or as a salt or a combination thereof,
and
a catalyst support selected from a group consisting of silica, alumina, and magnesium oxide, or a mixture thereof;
or
(ii) nickel, molybdenum, gold, and chromium, a catalyst support that is silica or alpha-alumina, and a promoter that is cesium;
and wherein the catalyst support is present in an amount that is equal to or greater than 80 weight percent based upon the total mass of all catalyst components.

2. The catalyst of Claim 1, wherein the promoter is cesium.

3. The catalyst of Claim 2, wherein the promoter further comprises calcium.

4. A process for producing one or more C₂-C₄ alcohols, which method comprises placing synthesis gas in contact with the catalyst of any of Claims 1-3 under conditions sufficient to convert at least a portion of the synthesis gas to at least one of ethanol, propanol and butanol, wherein the conversion temperature is from 200 to 500°C.

5. The process for producing C₂-C₄ alcohols according to Claim 4, wherein the catalyst is reduced using a reducing agent prior to contact with the synthesis gas.

6. The process for producing C₂-C₄ alcohols according to Claims 4 or 5, wherein at least a portion of the synthesis gas is converted to methanol.

7. The process for producing C₂-C₄ alcohols according to Claims 4 or 5, wherein at least a portion of the synthesis gas is converted to acetaldehyde.

8. The process for producing C₂-C₄ alcohols according to Claims 4 or 5, wherein the conditions include a minimum pressure of 500 psig (3.4 MPa).

## Patentansprüche

1. Ein Katalysator zur Umwandlung von Synthesegas, der Folgendes beinhaltet:
(i) Nickel, Molybdän, mindestens ein Metall, das aus einer Gruppe bestehend aus Palladium, Ruthenium, Chrom, Gold, Zirconium und Aluminum, ausgewählt ist;
wobei das mindestens eine Metall eine Kombination aus Palladium und Aluminum oder Ruthenium und Chrom oder Gold und Aluminum beinhaltet, einen Promotor, der mindestens eines von einem Alkalimetall oder einem Erdalkalimetall beinhaltet, wobei jeder Promotor als ein Metall, ein Hydroxid, ein Nitrid, ein Karbid oder als ein Salz oder eine Kombination davon vorhanden ist,
und
einen Katalysatorträger, der aus einer Gruppe, bestehend aus Siliciumdioxid, Aluminiumoxid und Magnesiumoxid oder einer Mischung davon, ausgewählt ist;
oder
(ii) Nickel, Molybdän, Gold und Chrom, einen Katalysatorträger, der Siliciumdioxid oder alpha-Aluminiumoxid ist, und einen Promotor, der Cäsium ist;
und wobei der Katalysatorträger in einer Menge vorhanden ist, die gleich oder größer ist als 80 Gewichtsprozent, bezogen auf die gesamte Masse aller Katalysatorkomponenten.

2. Katalysator gemäß Anspruch 1, wobei der Promotor Cäsium ist.

3. Katalysator gemäß Anspruch 2, wobei der Promotor ferner Calcium beinhaltet.

4. Ein Prozess zur Erzeugung von einem oder mehreren C₂-C₄-Alkoholen, wobei das Verfahren das In-Kontakt-Bringen von Synthesegas mit dem Katalysator gemäß einem der Ansprüche 1-3 unter Bedingungen beinhaltet, die ausreichend sind, um mindestens einen Teil des Synthesegases in mindestens eines von Ethanol, Propanol und Butanol umzuwandeln, wobei die Umwandlungstemperatur 200 bis 500 °C beträgt.

5. Prozess zur Erzeugung von C₂-C₄-Alkoholen gemäß Anspruch 4, wobei der Katalysator unter Verwendung von einem Reduktionsmittel vor dem Kontakt mit dem Synthesegas reduziert wird.

6. Prozess zur Erzeugung von C₂-C₄-Alkoholen gemäß Anspruch 4 oder 5, wobei mindestens ein Teil des Synthesegases in Methanol umgewandelt wird.

7. Prozess zur Erzeugung von C₂-C₄-Alkoholen gemäß Anspruch 4 oder 5, wobei mindestens ein Teil des Synthesegases in Acetaldehyd umgewandelt wird.

8. Prozess zur Erzeugung von C₂-C₄-Alkoholen gemäß Anspruch 4 oder 5, wobei die Bedingungen einen minimalen Druck von 500 psig (3,4 MPa) umfassen.

## Revendications

1. Un catalyseur de conversion de gaz de synthèse comprenant
(i) le nickel, le molybdène, au moins un métal sélectionné dans un groupe constitué du palladium, du ruthénium, du chrome, de l'or, du zirconium et de l'aluminium ; où ledit au moins un métal comprend une combinaison de palladium et d'aluminium, ou de ruthénium et de chrome, ou d'or et d'aluminium,
un promoteur comprenant au moins un métal parmi un métal alcalin ou un métal alcalinoterreux, où chaque promoteur est présent sous la forme d'un métal, d'un hydroxyde, d'un nitrure, d'un carbure, ou sous la forme d'un sel ou d'une combinaison de ceux-ci,
et
un support de catalyseur sélectionné dans un groupe constitué de la silice, de l'alumine, et de l'oxyde de magnésium, ou d'un mélange de ceux-ci ;
ou
(ii) le nickel, le molybdène, l'or, et le chrome, un support de catalyseur qui est la silice ou l'alumine-alpha, et un promoteur qui est le césium ;
et où le support de catalyseur est présent dans une quantité qui est égale ou supérieure à 80 pour cent en poids rapporté à la masse totale de tous les composants de catalyseur.

2. Le catalyseur de la revendication 1, où le promoteur est le césium.

3. Le catalyseur de la revendication 2, où le promoteur comprend en outre le calcium.

4. Un procédé pour produire un ou plusieurs alcools en C₂-C₄, laquelle méthode comprend le fait de placer du gaz de synthèse en contact avec le catalyseur de n'importe lesquelles des revendications 1 à 3 dans des conditions suffisantes pour convertir au moins une portion du gaz de synthèse en au moins un élément parmi l'éthanol, le propanol et le butanol, où la température de conversion va de 200 à 500 °C.

5. Le procédé pour produire des alcools en C₂-C₄ selon la revendication 4, où le catalyseur est réduit à l'aide d'un agent de réduction avant son contact avec le gaz de synthèse.

6. Le procédé pour produire des alcools en C₂-C₄ selon les revendications 4 ou 5, où au moins une portion du gaz de synthèse est convertie en méthanol.

7. Le procédé pour produire des alcools en C₂-C₄ selon les revendications 4 ou 5, où au moins une portion du gaz de synthèse est convertie en acétaldéhyde.

8. Le procédé pour produire des alcools en C₂-C₄ selon les revendications 4 ou 5, où les conditions incluent une pression minimale de 500 psig (3,4 MPa).
